(19) Europäisches Patentamt
European Patent Office
Office européen des brevets

(11) **EP 3 705 145 B1**

(12) **EUROPEAN PATENT SPECIFICATION**

(45) Date of publication and mention
of the grant of the patent:
**30.10.2024 Bulletin 2024/44**

(21) Application number: **17931049.5**

(22) Date of filing: **11.12.2017**

(51) International Patent Classification (IPC):
**A61M 1/36** *(2006.01)*   **A61M 1/38** *(2006.01)*
**G01N 21/17** *(2006.01)*   **A61M 1/34** *(2006.01)*

(52) Cooperative Patent Classification (CPC):
**A61M 1/3496; A61M 1/361; A61M 1/3621;**
**A61M 1/3672; A61M 1/38;** A61M 1/3693;
A61M 2202/0021; A61M 2202/0413;
A61M 2202/0415; A61M 2205/3306;
A61M 2230/207

(86) International application number:
**PCT/CN2017/115387**

(87) International publication number:
**WO 2019/085156 (09.05.2019 Gazette 2019/19)**

(54) **PLASMA COLLECTING SYSTEM FOR OPTIMIZING AMOUNT OF ANTICOAGULANT**

PLASMASAMMELSYSTEM ZUR OPTIMIERUNG DER ANTIKOAGULANSMENGE

SYSTÈME DE COLLECTE DE PLASMA PERMETTANT D'OPTIMISER LA QUANTITÉ D'ANTICOAGULANT

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **31.10.2017 CN 201711048620**

(43) Date of publication of application:
**09.09.2020 Bulletin 2020/37**

(73) Proprietor: **Sichuan Nigale Biotechnology Co., Ltd Chengdu, Sichuan 641403 (CN)**

(72) Inventors:
• **PENG, Chun**
**Chengdu**
**Sichuan 641403 (CN)**
• **WANG, Chaofu**
**Chengdu**
**Sichuan 641403 (CN)**

(74) Representative: **Ipside**
**7-9 Allée Haussmann**
**33300 Bordeaux Cedex (FR)**

(56) References cited:
EP-A1- 0 257 755   WO-A2-02/43806
CN-A- 104 800 905   CN-A- 104 800 905
CN-A- 105 498 010   CN-U- 204 395 097
US-A- 5 676 645   US-A1- 2015 314 060

EP 3 705 145 B1

## Description

## Technical Field

The present invention relates to the field of plasma collection and transfusion, in particular to a plasma collecting system for optimizing amount of anticoagulant.

## Background of the Invention

The plasmapheresis stations in China collect plasma by means of plasmapheresis machines at a uniform 1:16 anticoagulant to blood ratio. The main basis for establishing the ratio is that the general Hct ranges from 0.35 to 0.55 despite Hct varies among individuals. According to the ratio, the anticoagulation effect is ensured for all plasma donors with hematocrits (Hcts) in the normal range if the exact Hct of an individual is unknown before plasmapheresis. It also means that for people with higher Hct, in particular to males, the excessive ratio of the anticoagulant indicates an excessive anticoagulant dosage resulting in excessive citrate, and the probability of side effects caused by the excessive citrate increases accordingly. Therefore, optimization of the anticoagulant dosage reduces the impact of the anticoagulant on the health of long-term plasma donors.

Patent document CN 104 800 905 A discloses an anticoagulant control system and an anticoagulant control method for plasma collection. Patent document US 5 676 645 A discloses a method and apparatus for maximizing the total amount of blood processed during an apheresis procedure by optimizing the concentration of anticoagulant in a donor/patient and the associated extracorporeal tubing set. Patent document WO 02/43806 A2 discloses methods for determining and utilizing the viscosity of the circulating blood of a living being over a range of shear rates for diagnostics and treatment.

## Summary of the Invention

To solve the problem, the present invention provides a plasma collecting system for optimizing amount of anticoagulant according to the blood HCT content.

As a plasma collecting system for optimizing amount of anticoagulant, an embodiment of the invention comprises:

   a blood pump for driving blood to flow in the tube;
   an anticoagulant pump for driving an anticoagulant to flow in the tube;
   an HCT testing device for testing the blood HCT content;
   a calculating device for calculating a mixing ratio of the anticoagulant to whole blood according to the blood HCT content; and
   a control device for calculating the rotational speed ratio of an anticoagulant pump to a blood pump according to the mixing ratio of the anticoagulant to whole blood and a flow ratio of the anticoagulant pump to the blood pump.

Further, according to the blood HCT content, the mixing ratio of the anticoagulant to the whole blood is calculated as follows:

$$\frac{V_{ci}}{V_B} = \frac{A\,(1-HCT)}{A(1-HCT)+a},$$

Where, $A=P_{plasma}*C_{ci.min}$, $a=P_{plasma}*C_{Naci}$, $V_{ci}$ indicates the total amount of the anticoagulant required, $V_B$ indicates the total amount of the whole blood collected, $P_{plasma}$ indicates the average whole blood separation rate in the plasma collection process, $c_{ci.min}$ indicates the citrate concentration of the plasma, and $C_{Naci}$ indicates the concentration of 4% sodium citrate-based anticoagulant used by the plasmapheresis stations.

Further, the HCT testing device is embedded in a transparent tube connected with a blood collector and a plasma tube respectively at two ends.

Further, the HCT testing device comprises:

   a transmitter optical sub-assembly for transmitting an optical signal;
   a photosensitive receiving assembly for converting the optical signal passing through the transparent blood tube into an electrical signal;

a signal processing module comprising an amplifier and an AD converter; and

a microprocessor for calculating an HCT value according to the electric signal processed by the signal processing module.

Other aspects and advantages of the invention will become apparent from the following detailed description in combination with the drawings which illustrate, by way of embodiments, the principles of the invention.

## Brief description of the Drawings

The invention will be described in combination with embodiments and accompanying drawings, in which:

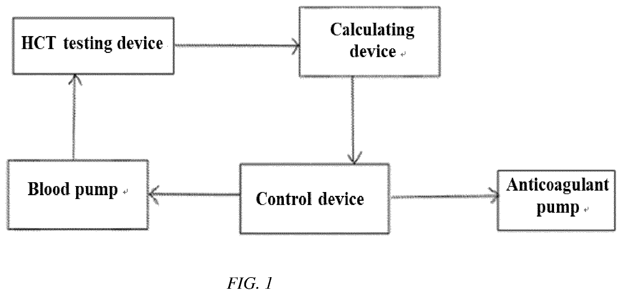

Fig. 1 shows a structural block diagram of a plasma collecting system for optimizing amount of anticoagulant provided by the embodiment of the invention.

## Detailed Description of Embodiments

All features or steps in all methods and procedures disclosed in the specification can be combined in any way, except mutually exclusive features and/or steps.

Any feature disclosed in the specification can be replaced with other equivalent or similar features, unless otherwise specified, that is, each feature is only an example of series of equivalent or similar features, unless otherwise specified.

Defects of the prior art: The anticoagulant ratio used for plasma collection in domestic plasmapheresis stations is uniformly set to 1:16. For people with high HCT, in particular to males, the anticoagulant dosage calculated according to a fixed ratio is excessively high. Part of the anticoagulant will enter the plasma donor with erythrocytes during the plasmapheresis, and the probability of side effects caused by the excessive citrate increases accordingly.

To solve the technical problem, the present invention provides a plasma collection system with highly-accurate anticoagulant dosage.

A plasma collecting system for optimizing amount of anticoagulant, comprises:

a blood pump for driving blood to flow in the tube;

an anticoagulant pump for driving an anticoagulant to flow in the tube;

an HCT testing device for testing the blood HCT content;

a calculating device for calculating a mixing ratio of the anticoagulant to whole blood according to the blood HCT content; and

a control device for calculating the rotational speed ratio of an anticoagulant pump to a blood pump according to the mixing ratio of the anticoagulant to whole blood and a flow ratio of the anticoagulant pump to the blood pump.

The anticoagulant pump of the invention is arranged in the anticoagulant tube to drive the anticoagulant to flow therein, and the blood pump is arranged in the blood tube to drive blood to flow therein. The anticoagulant pump and the blood pump are conventional devices for plasma collection. The anticoagulant pump and the blood pump work simultaneously to mix the anticoagulant and the blood and drive the mixture to flow into a centrifuge. The blood pump runs independently to drive the erythrocytes centrifugalized by the centrifuge back to the human body. In that case, part of the anticoagulant will enter the plasma donor with erythrocytes. Therefore, it is necessary to optimize the anticoagulant dosage to reduce the impact of the citrate-based anticoagulant on the health of the plasma donor.

The HCT testing device of the invention is embedded in a transparent tube connected with a blood collector and a plasma tube respectively at two ends. The HCT testing device comprises a transmitter optical sub-assembly for transmitting an optical signal, a photosensitive receiving assembly for converting the optical signal passing through the transparent blood tube into an electrical signal; a signal processing module comprising an amplifier and an AD converter; and a microprocessor for calculating an HCT value according to the electric signal processed by the signal processing module.

The blood drawn out by the blood collector passes through the transparent tube to form a transparent blood tube. When the optical signal from the transmitter optical sub-assembly irradiates the transparent blood tube, the intensity of the optical signal changes. The photosensitive receiving assembly converts the optical signal with changed intensity after irradiation to the transparent blood tube into the electrical signal. The electrical signal is amplified by a low-noise high-gain pre-amplifier and then input to an AD converter after an anti-aliasing filtering. The AD converter transmits the electrical signal to the microprocessor to analyze the electrical signal. An HCT value is obtained according to the obtained blood transmittance, and a blood HCT content is obtained accordingly. The HCT testing device regularly outputs the testing results through a serial interface. Only one external interface is arranged in the HCT testing device, and updates of communication, power supply and firmware are completed therein. The non-intrusive method of testing the blood HCT content introduces no contamination to the plasmapheresis and provides reliable basis for precision control of the anticoagulant dosage. In addition, the HCT testing device of the invention has advantages of low cost, simple operation and short testing time.

The calculating device of the invention is used to calculate the mixing ratio of the anticoagulant to whole blood by means of mathematical operations with the HCT content tested by the HCT testing device. The mixing ratio of the anticoagulant to whole blood refers to the volume ratio of the anticoagulant to whole blood in their mixture. The mixing ratio of the anticoagulant to whole blood is calculated as follows:

$$\frac{V_{ci}}{V_B} = \frac{A\,(1-HCT)}{A(1-HCT)+a},$$

Where, $A=P_{plasma}*C_{ci.min}$, $a=P_{plasma}*C_{Naci}$, $V_{ci}$ indicates the total amount of the anticoagulant required, $V_B$ indicates the total amount of whole blood collected, $P_{plasma}$ indicates the average whole blood separation rate in the plasma collection process, $C_{ci.min}$ indicates the citrate concentration of the plasma, and $C_{Naci}$ indicates the concentration of 4% sodium citrate-based anticoagulant used by the plasmapheresis stations.

Theoretical analysis of the anticoagulant dosage

1.1 Known conditional parameters:

1). a content of calcium ions ($C_{iCa}++$) in serum, unchanged with a content of serum protein;
2). a citrate concentration ($C_{ci.min}$) of plasma, sufficient to realize an effective anticoagulation effect;
3). Hematocrit (HCT)
4). in whole blood, the calcium ion content rises with the decreasing hematocrit (HCT); and
5). the concentration of 4% sodium citrate-based anticoagulant used by the plasmapheresis station ($C_{Naci}$) is 136mmol/L.

1.2 Calculation of anticoagulant ratio

1). $V_t$-target amount of plasma collected;
2). $P_{plasma}$-average whole blood separation rate in the process of plasma collection (refers to the percentage of plasma collected in all plasma in the whole blood capacity in a collection process; the Pplasma is used to measure the sufficiency of plasma separation and collection in a collection process).

The volume of citrate in the plasma is as follows:

$$V_{Ci.min} = V_t \times C_{ci.min} \qquad (1)$$

The volume of the anticoagulant in the plasma bag is as follows:

$$V_{PCi} = \frac{V_{ci.min}}{C_{NaCi}} \qquad (2)$$

The total amount of the anticoagulant required in the whole collection process is as follows:

$$V_{Ci} = \frac{V_{PCi}}{P_{plasma}} \qquad (3)$$

The relationship between the HCT and the anticoagulant ratio $R_{ac}$ is calculated as follows.

$$R_{ac} = \frac{\text{Total capacity of whole blood}}{\text{Total amount of the anticoagulant}}$$

$$= \frac{\text{Total plasma amount/ratio of plasma to whole blood}}{\text{Total amount of the anticoagulant}}$$

$$= \frac{\text{Total plasma /(1- HCT)}}{\text{Total amount of the anticoagulant}}$$

$$= \frac{\text{(Target collection amount/centrifugal separation rate; )/ (1-HCT)}}{\text{Total amount of the anticoagulant}} \qquad (4)$$

These formulas are simplified as follows:

$$R_{ac} = \frac{\left(V_t/P_{plasma}\right)/\left(1-Hct\right)}{V_{ci}}$$

$$= \frac{V_t}{P_{plasma} \times \left(1-Hct\right) \times V_{ci}}$$

$$= \frac{V_t}{\left(1-Hct\right) \times V_{PCi}}$$

$$= \frac{C_{NaCi}}{\left(1-Hct\right) \times C_{Ci.min}} \qquad (5)$$

Considering the errors in actual use, to ensure the anticoagulation effect, 1 is added to the minimum anticoagulant citrate concentration $C_{ci.min}$, i.e., $C_{ci.min}$=11 mmol/L.

The total whole blood capacity in the collection process is as follows:

$$V_B = \frac{V_t/P_{plasma}}{\left(1-Hct\right)}$$

$$= \frac{V_t}{P_{plasma} \times \left(1-Hct\right)} \qquad (6)$$

1.3 Calculation of theoretical minimum anticoagulant dosage

When calculating the theoretical minimum anticoagulant dosage, the new anticoagulant ratio $R_{ac}$ calculated by formula (5) is used

$$V_{Ci} = \frac{V_B}{R_{ac}+1}$$

$$= \frac{V_t}{P_{plasma} \times \left(1-Hct\right) \times \left(R_{ac}+1\right)} \qquad (7)$$

to obtain:

$$\frac{V_{ci}}{V_B} = \frac{A\ (1-HCT)}{A(1-HCT)+a},$$

Where,

$$A=P_{plasma}*C_{ci.min}, \quad a=P_{plasma}*C_{Naci},$$

$P_{plasma}$ indicates the average whole blood separation rate in the plasma collection process, and refers to the percentage of plasma collected in all plasma in the whole blood capacity in a collection process; and the $P_{plasma}$ is used to measure the sufficiency of plasma separation and collection in a collection process. The existing plasmapheresis stations adopt plasmapheresis machines at a fixed centrifugal speed. If a plasmapheresis machine at a centrifugal speed of 7000RPM is adopted as an embodiment, the centrifugal separation rate is about 70%, and the $P_{plasma}$ is 0.7. $C_{ci.min}$ indicates the citrate concentration of plasma. Considering the errors in actual use, to ensure the anticoagulation effect, 1 is added to the minimum anticoagulant citrate concentration $C_{ci.min}$, i.e., $C_{ci.min}$=11 mmol/L. $C_{Naci}$ indicates the concentration of 4% sodium citrate-based anticoagulant used by the plasmapheresis stations, $C_{Naci}$= 136 mmol/L. The HCT value obtained by the HCT testing device can be substituted into the formula to obtain a high-precision anticoagulant dosage. The required anticoagulant dosage is calculated by means of measuring the blood HCT content of every plasma donor to obtain the optimal anticoagulant dosage. The optimization is beneficial to the health of the plasma donor and to improving the quality of collected plasma.

The control device of the invention is used to calculate the rotational speed ratio of the anticoagulant pump to the blood pump according to the mixing ratio of the anticoagulant to whole blood and a flow ratio of the anticoagulant pump to the blood pump, and the implementations of the control device comprise but are not limited to a single-chip microcomputer, a microprocessor or a central processing unit. The rotational speed ratio of the anticoagulant pump to the blood pump is as follows:

$$\frac{V_N}{V_X} = \frac{V_{ci}}{V_B}*\frac{T_N}{T_X}$$

Where, $\frac{T_N}{T_X}$ indicates the flow ratio of the anticoagulant pump to the blood pump. Preferably, a flow monitoring device is arranged in the invention, to maintain the flow ratio of the anticoagulant pump and the blood pump at a set point.

The system of the invention further comprises an air detection probe respectively arranged in the anticoagulant tube and the blood tube to detect presence of air in the tube. The air detection probe determines the existence of air in the blood and the anticoagulant based on transfer velocity difference between ultrasonic in liquid and gas.

The various aspects, embodiments, implementations or features of the invention can be used separately or in any combination.

The invention has a plurality of advantages. Different aspects, embodiments or implementations yield one or more of the following advantages. The invention has an advantage that the required anticoagulant dosage is calculated by means of measuring the blood HCT content of every plasma donor to obtain the optimal anticoagulant dosage. The optimization is beneficial to the health of the plasma donor and to improving the quality of collected plasma. The invention has another advantage that the non-intrusive method of testing the blood HCT content introduces no contamination to the plasmapheresis and provides reliable basis for precision control of the anticoagulant dosage. The invention has another advantage that the HCT testing device of the invention is featured by low cost, short measurement time and simple operation, and the HCT testing device is simply clamped on a specified testing section of the consumables for measurement.

## Claims

1. A plasma collecting system for optimizing amount of anticoagulant, comprising:

    a blood pump for driving blood to flow in the tube;

an anticoagulant pump for driving an anticoagulant to flow in the tube;
an HCT testing device for testing the blood HCT content, HCT being referred to hematocrits;
a calculating device for calculating a mixing ratio of the anticoagulant to whole blood according to the blood HCT content; and
a control device for calculating the rotational speed ratio of an anticoagulant pump to a blood pump according to the mixing ratio of the anticoagulant to whole blood and a flow ratio of the anticoagulant pump to the blood pump, **characterized in that** the mixing ratio of the anticoagulant to whole blood is calculated as follows according to the blood HCT content:

$$\frac{V_{ci}}{V_B} = \frac{A\ (1-HCT)}{A(1-HCT)+a},$$

wherein, A=$P_{plasma}$*$C_{ci,min}$, a=$P_{plasma}$*$C_{Naci}$, $V_{ci}$ indicates the total amount of anticoagulant required, $V_B$ indicates the total amount of whole blood collected, $P_{plasma}$ indicates the average whole blood separation rate during plasma collection, $C_{ci.min}$ indicates the citrate concentration of the plasma, and $C_{Naci}$ indicates the concentration of 4% sodium citrate-based anticoagulant used by plasmapheresis stations.

2.  The plasma collecting system for optimizing amount of anticoagulant of claim 1, **characterized in that**:

the HCT testing device is embedded in a transparent tube connected with a blood collector and a plasma tube respectively at two ends.

3.  The plasma collecting system for optimizing amount of anticoagulant of claim 1, **characterized in that** the HCT testing device comprises:

a transmitter optical sub-assembly for transmitting an optical signal;
a photosensitive receiving assembly for converting the optical signal passing through the transparent blood tube into an electrical signal;
a signal processing module comprising an amplifier and an AD converter; and
and a microprocessor for calculating an HCT value according to the electric signal processed by the signal processing module.

4.  The plasma collecting system for optimizing amount of anticoagulant of claim 1, **characterized in that** the rotational speed ratio of the anticoagulant pump to the blood pump is:

$$\frac{V_N}{V_X} = \frac{V_{ci}}{V_B} * \frac{T_N}{T_X}$$

wherein, $\frac{T_N}{T_X}$ indicates the flow ratio of the anticoagulant pump to the blood pump.

5.  The plasma collecting system for optimizing amount of anticoagulant according to claim 1, **characterized in that** the plasma collecting system for optimizing amount of anticoagulant comprises an air detection probe respectively arranged in an anticoagulant tube and a blood tube to detect presence of air in the tube.

**Patentansprüche**

1.  Plasmasammelsystem zum Optimieren der Antikoagulansmenge, umfassend:

eine Blutpumpe zum Antreiben von Blut so, dass es in den Schlauch fließt;
eine Antikoagulanspumpe zum Antreiben eines Antikoagulans so, dass es in den Schlauch fließt;
eine HCT-Testvorrichtung zum Testen des Blut-HCT-Gehalts,
wobei sich HCT auf Hämatokrit bezieht;
eine Rechenvorrichtung zum Berechnen eines Mischungsverhältnisses des Antikoagulans zu Vollblut gemäß dem Blut-HCT-Gehalt; und

eine Steuervorrichtung zum Berechnen des Drehzahlverhältnisses einer Antikoagulanspumpe zu einer Blutpumpe gemäß dem Mischungsverhältnis des Antikoagulans zu Vollblut und einem Strömungsverhältnis der Antikoagulanspumpe zur Blutpumpe,

**dadurch gekennzeichnet, dass** das Mischungsverhältnis des Antikoagulans zu Vollblut wie folgt gemäß dem Blut-HCT-Gehalt berechnet wird:

$$\frac{V_{ci}}{V_B} = \frac{A\ (1-HCT)}{A(1-HCT)+a},$$

wobei $A=P_{plasma}*C_{ci.min}$, $a=P_{plasma}*C_{Naci}$, $V_{ci}$ die erforderliche Gesamtmenge an Antikoagulans angibt, $V_B$ die gesammelte Gesamtmenge an Vollblut angibt, $P_{plasma}$ die durchschnittliche Vollblut-Abscheiderate während Plasmasammlung angibt, $C_{ci.min}$ die Citratkonzentration des Plasmas angibt, und $C_{Naci}$ die Konzentration von Antikoagulans auf Basis von 4 % Natriumcitrat angibt, das von Plasmapherese-Stationen verwendet wird.

2. Plasmasammelsystem zum Optimieren der Antikoagulansmenge nach Anspruch 1, **dadurch gekennzeichnet, dass**:
   die HCT-Testvorrichtung in einen transparenten Schlauch eingebettet ist, der an zwei Enden mit einem Blutsammler bzw. einem Plasmaschlauch verbunden ist.

3. Plasmasammelsystem zum Optimieren der Antikoagulansmenge nach Anspruch 1, **dadurch gekennzeichnet, dass** die HCT-Testvorrichtung umfasst:

   eine optische Sender-Unterbaugruppe zum Senden eines optischen Signals;
   eine lichtempfindliche Empfangsbaugruppe zum Umwandeln des optischen Signals, das durch den transparenten Blutschlauch tritt, in ein elektrisches Signal;
   ein Signalverarbeitungsmodul, das einen Verstärker und
   einen AD-Wandler umfasst; und
   einen Mikroprozessor zum Berechnen eines HCT-Wertes gemäß dem vom Signalverarbeitungsmodul verarbeiteten elektrischen Signal.

4. Plasmasammelsystem zum Optimieren der Antikoagulansmenge nach Anspruch 1, **dadurch gekennzeichnet, dass** das Drehzahlverhältnis der Antikoagulanspumpe zur Blutpumpe ist:

$$\frac{V_N}{V_X} = \frac{V_{ci}}{V_B} * \frac{T_N}{T_X}$$

wobei $\frac{T_N}{T_X}$ das Strömungsverhältnis der Antikoagulanspumpe zur Blutpumpe angibt.

5. Plasmasammelsystem zum Optimieren der Antikoagulansmenge nach Anspruch 1, **dadurch gekennzeichnet, dass** das Plasmasammelsystem zum Optimieren der Antikoagulansmenge eine Lufterkennungssonde umfasst, die jeweils in einem Antikoagulansschlauch und einem Blutschlauch angeordnet ist, um das Vorhandensein von Luft im Schlauch zu erkennen.

**Revendications**

1. Système de collecte de plasma pour optimiser une quantité d'anticoagulant, comprenant :

   une pompe à sang pour amener du sang à s'écouler dans le tube ;
   une pompe à anticoagulant pour amener un anticoagulant à s'écouler dans le tube ;
   un dispositif de test HCT pour tester la teneur en HCT dans le sang, HCT faisant référence à des hématocrites ;
   un dispositif de calcul pour calculer un rapport de mélange entre l'anticoagulant et le sang total en fonction de la teneur en HCT dans le sang ; et

un dispositif de commande pour calculer le rapport de vitesse de rotation entre une pompe à anticoagulant et une pompe à sang en fonction du rapport de mélange entre l'anticoagulant et le sang total et d'un rapport de débit entre la pompe à anticoagulant et la pompe à sang,

**caractérisé en ce que** le rapport de mélange entre l'anticoagulant et le sang total est calculé de la manière suivante en fonction de la teneur en HCT dans le sang :

$$\frac{V_{ci}}{V_B} = \frac{A(1 - HCT)}{A(1 - HCT) + a}$$

où $A = P_{plasma} * C_{ci.min}$, $a = P_{plasma} * C_{Naci}$, $V_{ci}$ indique la quantité totale d'anticoagulant requis, $V_B$ indique la quantité totale de sang total collecté, $P_{plasma}$ indique le taux moyen de séparation du sang total lors de la collecte de plasma, $C_{ci.min}$ indique la concentration en citrate du plasma, et $C_{Naci}$ indique la concentration d'anticoagulant basé sur du citrate de sodium à 4 % utilisé par des stations de plasmaphérèse.

2. Système de collecte de plasma pour optimiser une quantité d'anticoagulant selon la revendication 1, **caractérisé en ce que** :
   le dispositif de test HCT est intégré dans un tube transparent relié à un collecteur de sang et à un tube de plasma, respectivement à deux extrémités.

3. Système de collecte de plasma pour optimiser une quantité d'anticoagulant selon la revendication 1, **caractérisé en ce que** le dispositif de test HCT comprend :

   un sous-ensemble optique émetteur pour émettre un signal optique ;
   un ensemble de réception photosensible pour convertir le signal optique passant à travers le tube de sang transparent en un signal électrique ;
   un module de traitement de signal comprenant un amplificateur et un convertisseur AN ; et
   un microprocesseur pour calculer une valeur HCT en fonction du signal électrique traité par le module de traitement de signal.

4. Système de collecte de plasma pour optimiser une quantité d'anticoagulant selon la revendication 1, **caractérisé en ce que** le rapport de vitesse de rotation entre la pompe à anticoagulant et la pompe à sang est :

$$\frac{V_N}{V_X} = \frac{V_{ci}}{V_B} * \frac{T_N}{T_X}$$

où, $\dfrac{T_N}{T_X}$ indique le rapport de débit entre la pompe à anticoagulant et la pompe à sang.

5. Système de collecte de plasma pour optimiser une quantité d'anticoagulant selon la revendication 1, **caractérisé en ce que** le système de collecte de plasma pour optimiser une quantité d'anticoagulant comprend une sonde de détection d'air disposée respectivement dans un tube d'anticoagulant et un tube de sang pour détecter la présence d'air dans le tube.

*FIG. 1*

**EP 3 705 145 B1**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- CN 104800905 A
- US 5676645 A

- WO 0243806 A2